# EUROPÄISCHE PATENTSCHRIFT

(11) **EP 3 288 475 B1**
(45) Veröffentlichungstag und Bekanntmachung des Hinweises auf die Patenterteilung: **31.10.2018**
(21) Anmeldenummer: 17722778.2
(22) Anmeldetag: 10.05.2017
(51) Int. Cl.: A61B 17/70, A61B 17/86

(54) **PEDIKELSCHRAUBE MIT KNOCHENGEWINDE VON GROSSEM DURCHMESSER**
PEDICLE SCREW WITH LARGE-DIAMETER BONE THREAD
VIS PÉDICULAIRE PRÉSENTANT UN FILETAGE À OS DE GRAND DIAMÈTRE

(30) Priorität: 13.05.2016 DE 102016108972
(43) Veröffentlichungstag der Anmeldung: 07.03.2018
(73) Patentinhaber: Aesculap AG, 78532 Tuttlingen (DE)
(72) Erfinder: LINDNER, Stephan, 78573 Wurmlingen (DE)
(74) Vertreter: Winter, Brandl, Fürniss, Hübner, Röss, Kaiser, Polte - Partnerschaft mbB
(86) Internationale Anmeldenummer: PCT/EP2017/061232
(87) Internationale Veröffentlichungsnummer: WO 2017/194633

(56) Entgegenhaltungen:
- DE-A1-102004 027 881
- GB-A- 2 512 063
- US-A1- 2008 015 596
- US-A1- 2012 215 263

## Beschreibung

Die vorliegende Erfindung betrifft ein Pedikelschraubensystem umfassend eine Aufnahmehülse für einen Längsträger, eine Knochenschraube und ein Schaftkopfelement, wobei die Knochenschraube ein als Außengewinde ausgebildetes Knochengewinde und ein als Innengewinde ausgebildeten Verbindungsgewinde aufweist, wobei das Schaftkopfelement proximal (aus der Sicht des Chirurgen) einen Kopf, auf dem die Aufnahmehülse gelagert ist, und distal (aus der Sicht des Chirurgen) ein als Außengewinde ausgebildetes Verbindungsgewinde aufweist und damit in das Verbindungsgewinde der Knochenschraube eingeschraubt ist.

Pedikelschrauben dienen prinzipiell zur dorsalen Stabilisierung der Wirbelsäule bei Fakturen, Tumoren, Entzündungen, Deformitäten und degenerativ bedingten Instabilitäten mittels transpedikulärer Verschraubung. Dabei werden Pedikelschrauben in den Pedikeln jeweils benachbarter Wirbel platziert, worauf eine winkelstabile Verbindung zwischen den jeweils axial übereinander sich anordnenden Pedikelschrauben und einem axial sich erstreckenden Längsträger oder Steg geschaffen wird. Die Pedikelschrauben und Längsträger bilden dabei ein Wirbel-Stabilisierungssystem.

Hierfür hat eine Pedikelschraube in der Regel ein sich in axialer Richtung erstreckendes Außengewinde als Knochengewinde, an den sich schraubenkopfseitig eine Aufnahmehülse (sogenannte Tulpe) anschließt. Diese bildet konstruktiv eine U-förmig längs geschlitzte/getunnelte Aufnahme mit Innengewinde zur Aufnahme eines Längsträgers, wobei die beiden sich radial gegenüberliegenden Längsschlitze jeweils einen Schlitzspalt vorbestimmter Spaltbreite definieren. In die parallel zueinander verlaufenden Längsschlitze ist der Längsträger quer einlegbar und mittels eines Verriegelungselements, zum Beispiel in Form einer Madenschraube, Gewindemutter oder Setscrew, die in das Innengewinde eingedreht ist, fixierbar.

Bei einer polyaxialen Pedikelschraube ist ein zumeist kugelförmiger oder (semi-) sphärischer Schaftkopf von der Aufnahmehülse/Tulpe relativ verschwenkbar umgriffen und gleichzeitig im Übergangsbereich zwischen Kopf und Schaft hintergriffen. Auf diese Weise kann die Aufnahmehülse/Tulpe nach Versenken des Außengewindeschafts im Pedikelkanal eines Wirbels relativ dazu verschwenkt und/oder verdreht werden, um eine gewünschte Lage und Ausrichtung im Wesentlichen unabhängig zur Ausrichtung des Schafts zu erhalten. Die Hinterschneidung verhindert dabei, dass die Aufnahmehülse/Tulpe vom Schaftkopf abgezogen werden kann.

In der Praxis können Fälle auftreten, bei denen Pedikelschrauben mit einem Knochengewinde mit großem Durchmesser erforderlich sind. Insbesondere bei einer polyaxialen Pedikelschraube mit großen Knochenschraubendurchmesser kann problematisch sein, dass der maximal mögliche Knochenschraubendurchmesser durch die Geometrie und die Abmessungen der Aufnahmehülse bestimmt und begrenzt ist. Konstruktionsbedingt ist aber die Knochenschraube von proximal nach distal durch die Aufnahmehülse hindurchzuführen, so dass ihr Schaftkopf gelagert in der Aufnahmehülse zu liegen kommt. Grund ist hierbei die Forderung nach einem möglichst kleinen Kopfteil bzw. einer möglichst kleinen Aufnahmehülse und dem sich daraus ergebenden kleinen Innendurchmesser der Aufnahmehülse.

Gattungsgemäßer Stand der Technik ist etwa aus der DE 10 2004 027 881 A1 bekannt. Diese offenbart eine Knochenschraube mit einem im Wesentlichen einheitlichen Verbindungsgewindeabschnitt. Vergleichbare Vorrichtungen sind aus der US 2008 / 0015596 A1, der GB 2512063 A sowie der US 2012 / 0215263 A1 bekannt.

Um das vorstehend beschriebene Problem zu lösen, wurde vom Anmelder dieser Erfindung bereits ein Design für eine mehrteilige Pedikelschraube entwickelt, welches eine Knochenschraube und ein Schaftkopfelement aufweist. Die Knochenschraube weist ein als Außengewinde ausgebildetes Knochengewinde und ein als Innengewinde ausgebildeten Verbindungsgewinde auf. Das Schaftkopfelement weist wiederum proximal einen Kopf zur Lagerung der Aufnahmehülse und distal ein als Außengewinde ausgebildetes Verbindungsgewinde auf. Das Schaftkopfelement und die Knochenschraube sind über die Verbindungsgewinde miteinander verbunden. Durch diese zweiteilige Ausführung wird der Vorteil erzielt, dass das Schaftkopfelement auf die Abmessungen der Aufnahmehülse abgestimmt ausgebildet sein kann, also derart, dass es durch diese von proximal nach distal bis zum Anschlag am Schaftkopf hindurchgeführt werden kann, während die Knochenschraube aus distaler Richtung, ohne durch die Aufnahmehülse hindurch geführt werden zu müssen, auf das in die Aufnahmehülse vormontierte Schaftkopfelement aufgeschraubt werden kann und hinsichtlich seines Knochengewindedurchmesser nicht beschränkt ist.

Ein mögliches Problem bei dieser mehrteiligen Pedikelschraube kann in bestimmten Fällen sein, dass das Knochengewinde im Bereich der Verschraubung von Knochenschraube und Schaftkopfelement, also im Bereich der Verbindungsverschraubung, sehr flach und konisch vorgesehen werden muss, um eine ausreichende Stabilität der Knochenschraube sicherzustellen und um die Wahrscheinlichkeit eines Bruchs der Knochenschraube infolge der dünnen Wand zwischen dem Knochengewinde und dem Verbindungsgewinde begünstigt durch deren hohe Kerbwirkungen vermindern bzw. verhindern zu können. Es gibt jedoch Anwendungen, bei denen darauf Wert gelegt wird, dass die Knochenschraube an Stelle von einem konischen Gewindekern einen zylindrischen oder annähernd zylindrischen Gewindekern aufweist.

Ausgehend vom vorgenannten Stand der Technik liegt der Erfindung die Aufgabe zugrunde, ein Pedikelschraubensystem bereit zu stellen, das bei gleichem Außendurchmesser, gleicher Eindrehfestigkeit, gleicher Ausdrehfestigkeit und gleicher biomechanischen Dauerfestigkeit einen zylindrischen oder annähernd zylindrischen Gewindekern aufweist. Insbesondere soll eine möglichst große Gewindetiefe des Knochengewindes vorgesehen sein. Es soll eine Übertragung von Eindrehmomenten ≥ 12 Nm und eine Übertragung von Ausdrehmomenten ≥ 10 Nm möglich sein. Bei einem Versagen, insbesondere bei einem Bruch des Implantats, bei einer Revision oder bei einer ungewollten intraoperativen Demontage des Implantats soll ein Ausschrauben des Knochengewindeteils aus dem Knochen ohne wesentliche Probleme möglich sein. Schließlich ist zwischen der Knochenschraube und dem Schaftkopfelement eine dichte Verbindung erforderlich, um eine ausreichende Reinigungseignung zu gewährleisten.

Diese Aufgabe wird nach der vorliegenden Erfindung gelöst durch ein Pedikelschraubensystem nach dem Oberbegriff von Anspruch 1, wobei die Verbindungsgewinde jeweils als (vorzugsweise ausgerichtete / gleichlaufende) Mehrfachgewinde mit jeweils einem distalen Verbindungsgewindeabschnitt und einem proximalen Verbindungsgewindeabschnitt ausgebildet sind. Der distale Verbindungsgewindeabschnitt und der proximale Verbindungsgewindeabschnitt weisen eine identische oder gleiche Gewindesteigung auf. Der distale Verbindungsgewindeabschnitt besitzt einen kleineren Durchmesser, insbesondere einen kleineren Nenndurchmesser, als der proximale Verbindungsgewindeabschnitt.

Mittels der unterschiedlich großen Durchmesser des distalen Verbindungsgewindeabschnitts (klein / gering) und des proximalen Verbindungsgewindeabschnitts (groß / weit) werden die folgenden, gegenläufigen Effekte erreicht: Zum einen mindert der geringe distale Verbindungsgewindeabschnitt die auf den Knochen wirkende Kerbwirkung. Dies ermöglicht der Schraube, einer hohen Zugwiderstandskraft / "Pull-Out-Kraft" entgegenwirken zu können. Zum anderen ist mittels des weiten proximalen Verbindungsgewindeabschnitts eine erhöhte Momentenbelastung der Schraube möglich, welche ein gesteigertes Einstellmoment zulässt.

Um eine sichere, stabile und dauerhafte Verbindung von Knochenschraube und Schaftkopfelement sicherstellen zu können, ist eine bestimmte Länge des Verbindungsgewindes erforderlich. Nach der der Erfindung zugrunde liegenden Idee wird das Verbindungsgewinde zwischen Knochenschraube und Schaftkopfelement bekannter Pedikelschraubensysteme quasi in mehrere Gewindeabschnitte aufgeteilt. Deren Gewindedurchmesser, insbesondere Nenndurchmesser, unterscheiden sich jeweils voneinander. Proximale Verbindungsgewindeabschnitte besitzen einen größeren Durchmesser (Nenndurchmesser) als distale Verbindungsgewindeabschnitte. Man kann also sagen, dass die Durchmesser der Verbindungsgewindeabschnitte von proximal nach distal kleiner werden. Ein weiterer dadurch bewirkter Effekt ist, dass sich die Wandstärke der Knochenschraube im Bereich der Verbindungsgewinde stufenweise ändert, und zwar in mehreren gegenüber den Stand der Technik kleineren Schritten (dort gibt es am Verbindungsgewindeauslauf eine relativ große Querschnittsänderung mit entsprechender Kerbwirkung). Dadurch kann zum einen die am Ende des Verbindungsgewindes, insbesondere am distalen Verbindungsgewindeauslauf, auftretende Kerbwirkung gegenüber den Stand der Technik minimiert werden. Zum anderen wird die Kerbwirkung durch Vorsehen mehrerer Verbindungsgewindeausläufe, nämlich an jedem Verbindungsgewindeabschnitt, auf einen größeren Abschnitt der Knochenschraube in Längsrichtung verteilt, nämlich auf die gesamte Länge des Verbindungsgewindes. Insgesamt können durch die Erfindung die im Bereich des Verbindungsgewindes auf die Knochenschraube wirkenden Belastungen und eingebrachten Spannungen gegenüber dem Stand der Technik bei gleicher Gewindelänge wesentlich reduziert werden. Das erfindungsgemäße Pedikelschraubensystem kann daher bei gleichem Außendurchmesser, gleicher Eindrehfestigkeit, gleicher Ausdrehfestigkeit und gleicher biomechanischen Dauerfestigkeit mit (im Wesentlichen) zylinderförmigem Knochengewindekern ausgebildet werden.

Zur Klarstellung sei überdies erwähnt, dass eine "identische Gewindesteigung" des proximalen und distalen Verbindungsgewindeabschnitts im Sinne der Erfindung bedeutet, dass sich diese in ihrer Steigung nicht mehr als 10% zueinander unterscheiden, sodass die daraus (möglicherweise) resultierende Abweichung tolerabel ist, das heißt dass die Schraube bei jenen geringen Abweichungen eindrehbar bleibt.

Vorteilhafte Ausführungsformen der Erfindung sind in den Unteransprüchen beansprucht und werden nachfolgend näher erläutert.

Eine Ausführungsform der Erfindung ist dadurch gekennzeichnet, dass die Verbindungsgewinde der Knochenschraube als Doppelgewinde ausgebildet sind. Dieses weist einen ersten, distalen Verbindungsgewindeabschnitt und einen zweiten, proximalen Verbindungsgewindeabschnitt auf. Des Weiteren sind die Verbindungsgewinde des Schaftkopfelements als Doppelgewinde ausgebildet, ebenfalls mit einem ersten, distalen Verbindungsgewindeabschnitt und einem zweiten, proximalen Verbindungsgewindeabschnitt.

Alternativ oder zusätzlich können der Gewindedurchmesser und die Gewindesteigung jedes Verbindungsgewindeabschnitts jeweils konstant sein.

Es ist von besonderem Vorteil, wenn das Schaftkopfelement einen Anschlag aufweist, an dem eine Anschlagfläche der Knochenschraube, insbesondere deren proximale Stirnfläche anliegt, wenn das Schaftkopfelement und die Knochenschraube über ihre jeweiligen Verbindungsgewinde vollständig miteinander verschraubt sind. Das nach der Erfindung genutzte Mehrfachgewinde bewirkt gemeinsam mit dem Anschlag, dass die Knochenschraube im gesamten Bereich zwischen dem distalen Verbindungsgewinde und Anschlag unter Druckspannung gebracht wird. Diese Druckspannung bewirkt eine größere Kapazität hinsichtlich einer Aufnahme von die Knochenschraube wirkenden biomechanischen Kräften. Das Schaftkopfelement und die Knochenschraube können am Anschlag insbesondere dichtend aneinander anliegen, so dass das Verbindungsgewinde nicht verschmutzt und gut zu reinigen ist.

Eine Ausführungsform der Erfindung ist dadurch gekennzeichnet, dass die Knochenschraube eine sich in Richtung der Längsachse erstreckende zentrale, gestufte Öffnung aufweist, insbesondere in Form einer Sackbohrung oder einer Durchgangsbohrung. Die Öffnung kann mehrere Abschnitte aufweisen, deren Anzahl zumindest der Anzahl der Verbindungsgewindeabschnitte entspricht, wobei die Abschnitte zueinander unterschiedliche Innendurchmesser besitzen. Eine solche zentrale Öffnung erleichtert das Einschrauben des Schaftkopfelements in die Knochenschraube. Im Falle einer Durchgangsöffnung kann diese zum Beispiel genutzt werden, um eine Knochenverbindungsmaterial, zum Beispiel Knochenzement, durch die Schraube an den Knochen zu bringen.

Eine Ausführungsform der Erfindung ist dadurch gekennzeichnet, dass die zentrale Öffnung der Knochenschraube einen proximalen gewindelosen Abschnitt und distalen gewindelosen Abschnitt aufweist, zwischen denen der distale Verbindungsgewindeabschnitt und der proximale Verbindungsgewindeabschnitt angeordnet sind. Solche gewindelosen Abschnitte können in vorteilhafter Weise als Positionierungshilfen verwendet werden und erleichtern das Setzen der Pedikelschraube durch einen Operateur.

Nach einer Ausführungsform der Erfindung ist vorgesehen, dass das Schaftkopfelement einen gestuften Zapfen aufweist, mit einem proximalen gewindelosen Abschnitt und einem distalen gewindelosen Abschnitt, zwischen denen der distale Verbindungsgewindeabschnitt und der proximale Verbindungsgewindeabschnitt angeordnet sind.

Es ist von besonderem Vorteil, wenn der distale gewindelose Abschnitt einen kleineren Durchmesser aufweist als der distale Verbindungsgewindeabschnitt und dass der proximale gewindelose Abschnitt einen größeren Durchmesser aufweist als der proximale Verbindungsgewindeabschnitt. Hierdurch kann Kerbwirkung infolge von Querschnittänderungen reduziert oder gering gehalten werden.

Eine weitere Ausführungsform der Erfindung ist dadurch gekennzeichnet, dass das Knochengewinde einen zylinderförmigen oder im Wesentlichen oder annähernd zylinderförmigen Gewindekern mit im Wesentlichen konstantem Kerndurchmesser aufweist. Ein solches Knochengewinde sichert einen guten Halt der Knochenschraube im Knochen und eine gleichmäßige Lasteinbringung über die gesamte Länge des Knochengewindes in das Knochenmaterial. Eine weitere Ausführungsform der Erfindung ist dadurch gekennzeichnet, dass die Gewindetiefe des Knochengewindes im Bereich der Verbindungsgewinde konstant ist.

Nach einer Ausführungsform kann der proximale gewindelose Abschnitt einen Torxprofilaufnahmeabschnitt zur Aufnahme eines Torxschraubendrehers aufweisen, um eine Revision der Knochenschraube zu ermöglichen, falls sich das Schaftkopfelement von der Knochenschraube gelöst hat, oder ein unmittelbares (nicht über das Schaftkopfelement) Einschrauben oder Herausschrauben der Knochenschraube zu ermöglich.

In einer alternativen Ausführungsform ist der proximale Verbindungsgewindeabschnitt zum distalen Verbindungsgewindeabschnitt innerhalb eines gewissen Toleranzbereichs in der Umfangsrichtung der Schraube versetzt. Hieraus resultiert beim Einschrauben der Schraube eine gewisse Verspannung, welche das Festsitzen / die aufzubringende Pull-Out-Kraft (ebenso wie etwaige Abweichungen in der Gewindesteigung) der Schraube begünstigt.

Zusammenfassend kann man sagen, dass durch die Erfindung eine zweiteilige Knochenschraube ermöglicht wird, welche zwei oder mehrere Gewindeabschnitte aufweist, welche zueinander (im Wesentlichen) ausgerichtet sind und die (im Wesentlichen) gleiche Gewindesteigung aufweisen. Der Gewindedurchmesser kann hierbei von vorne nach hinten abnehmen. Man kann insbesondere sagen, dass durch die Erfindung ein polyaxiales Pedikelschraubensystem mit mehrteiliger, insbesondere zweiteiliger, Knochenschraube ermöglicht wird, bei dem die Knochenschraube bei gleichem Außendurchmesser wie eine Knochenschraube mit konischem Knochengewinde nach dem Stand der Technik ein im Wesentlichen zylindrisches Gewindekernprofil mit ausgeprägter Gewindetiefe aufweist.

Weitere Merkmale und Vorteile der vorliegenden Erfindung ergeben sich aus der folgenden beispielhaften und nicht beschränkenden Beschreibung der Erfindung anhand von Figuren. Diese sind lediglich schematischer Natur und dienen nur dem Verständnis der Erfindung. Dabei zeigen:
Fig. 1 in einer schematischen Schnittdarstellung ein Pedikelschraubensystem nach dem Stand der Technik und
Fig. 2 in einer schematischen Schnittdarstellung ein Pedikelschraubensystem nach der Erfindung.

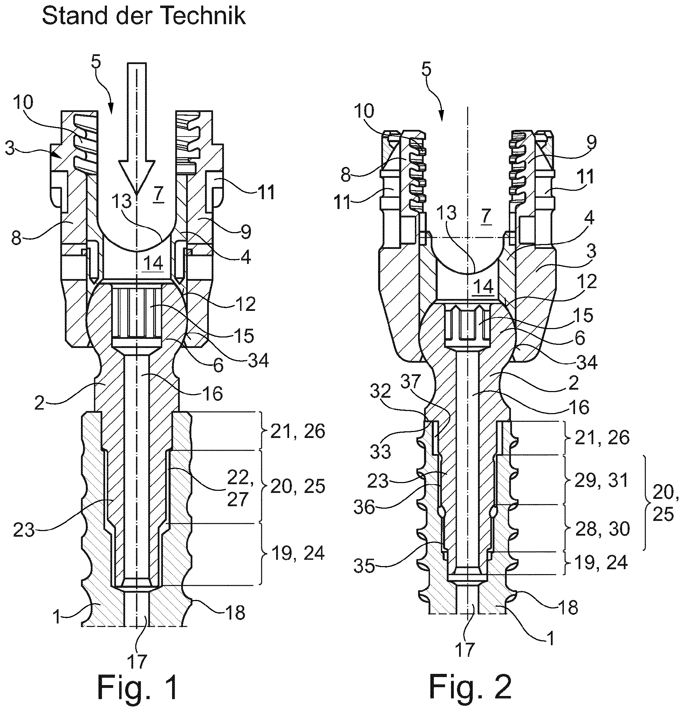

Beide Pedikelschraubensysteme, das nach dem Stand der Technik in Figur 1 wie auch das nach der Erfindung in Figur 2, umfassen eine Knochenschraube 1, ein Schaftkopfelement 2, eine Aufnahmehülse 3, auch als Tulpe 3 bezeichnet und einen Klemmstempel 4.

Die Aufnahmehülse 3 ist als gesondertes Bauteil ausgebildet und beweglich am Schaftkopfelement 2 angeordnet, so dass ein polyaxiales Pedikelschraubensystem ausgebildet ist, bei dem die Aufnahmehülse 3 relativ zur Knochenschraube 1 und zum Schaftkopfelement 2 winkelpositionierbar, insbesondere drehbar und/oder schwenkbar, ist. Sie weist eine im Wesentlichen zylinderförmige Grundgestalt mit in einer Durchgangsöffnung 5 in Längsrichtung auf. Der distale Endbereich der Durchgangsöffnung 5 ist sich in radialer Richtung kugelabschnittsförmig nach innen verjüngend ausgebildet und bildet eine Kugelaufnahme 34 für einen Kugelkopf 6 des Schaftkopfelements 2 aus. Proximal ist in der Aufnahmehülse 3 ein Aufnahmeraum 7 für einen in den Figuren nicht dargestellten Längsträger ausgebildet. Dieser ist seitlich von zwei radial einander gegenüberliegenden Hülsenwandabschnitten 8, 9 auf, in deren einander zugewandten Innenflächen ein Innengewinde 10 eingebracht ist. Dieses dient der Aufnahme einer in den Figuren ebenfalls nicht gezeigten Klemmschraube, zum Beispiel in Form einer üblichen Madenschraube. In die Außenseiten der Hülsenwandabschnitte 8, 9 ist jeweils eine Werkzeugaufnahme 11 eingebracht, zur Aufnahme und Kopplung eines medizintechnischen Instruments zum Handhaben der Aufnahmehülse 3 oder des gesamten Pedikelschraubensystems.

In der Durchgangsöffnung 5 ist der Klemmstempel 4 angeordnet. Er liegt in radialer Richtung an der die Durchgangsöffnung 5 umgebenden Hülsenwandung an und weist an seinem distalen Ende eine teilkugelförmige Aufnahmefläche 12 zur klemmenden Anlage am Kugelkopf 6 auf. Sein proximales Ende ist zu einer etwa halbschalenförmigen Lagerschale 13 für den Längsträger ausgebildet. Er weist eine zentrale Durchgangsöffnung 14 auf.

Das Schaftkopfelement 2 weist an seinem proximalen Ende den Kugelkopf 6 auf. In diesen ist eine Werkzeugaufnahme 15, hier in Form eines Innensechskants, eingebracht. Auch das Schaftkopfelement 2 ist mit einer zentralen Durchgangsöffnung 16 in Richtung der Längsachse versehen.

Die Knochenschraube 1 weist ebenfalls eine zentrale Durchgangsöffnung 17 in Richtung der Längsachse auf. An ihrer radial äußeren Seite ist sie mit einem Knochengewinde 18 versehen. In den proximalen Endbereich der Knochenschraube 1 ist die Durchgangsöffnung 17 in radialer Richtung aufgeweitet.

Bei der Knochenschraube 1 nach dem Stand der Technik ist in der Durchgangsöffnung 17 ein distaler aufgeweiteter Abschnitt 19, ein medialer aufgeweiteter Abschnitt 20 und ein proximaler aufgeweiteter Abschnitt 21 ausgebildet. Der mediale aufgeweitete Abschnitt 20 ist mit einem Verbindungsgewinde 22 versehen. Der Innendurchmesser des distalen aufgeweiteten Abschnitts 19 ist kleiner als der des medialen aufgeweiteten Abschnitts 20, der wiederum kleiner ist als der des proximalen aufgeweiteten Abschnitts 21. Anders ausgedrückt steigt der Innendurchmesser von distal nach proximal an. Das Schaftkopfelement 2 nach dem Stand der Technik weist distal einen Schaft 23 auf. Dieser ist gestuft ausgebildet, mit einem distalen Schaftabschnitt 24, einem medialen Schaftabschnitt 25 und einem proximalen Schaftabschnitt 26. Der mediale aufgeweitete Schaftabschnitt 25 ist mit einem Verbindungsgewinde 27 versehen. Der Innendurchmesser des distalen aufgeweiteten Schaftabschnitts 24 ist kleiner als der des medialen aufgeweiteten Schaftabschnitts 25, der wiederum kleiner ist als der des proximalen aufgeweiteten Schaftabschnitts 26. Anders ausgedrückt steigt der Innendurchmesser von distal nach proximal an. Die beiden Verbindungsgewinde 22, 27 stehen miteinander in Gewindeeingriff und koppeln die Knochenschraube 1 mit dem Schaftkopfelement 2. Durch diese Konstruktion ist das Schaftkopfelement 2 von proximal nach distal durch die Aufnahmehülse 3 einsetzbar und kann dann mit der Knochenschraube 1 verschraubt werden, so dass diese mit nahezu beliebigem Nenndurchmesser unabhängig von der Geometrie der Aufnahmehülse 3 ausgebildet sein kann. Nachteil der in Figur 1 gezeigten Version nach dem Stand der Technik ist, dass die Knochenschraube 1 in Höhe des Übergangs vom medialen aufgeweiteten Abschnitt 20 zum distalen aufgeweiteten Abschnitt 19 infolge der dort konzentriert auftretenden Kerbwirkung bevorzugt brechen kann, da hier, bedingt durch das Knochengewinde die Wandstärke sehr gering ist und damit ein großer Spannungssprung vorhanden ist.

Dieses Problem wird durch die Erfindung gelöst, die in Figur 2 gezeigt ist. Auch die Knochenschraube 1 nach der Erfindung ist in der Durchgangsöffnung 17 ein distaler aufgeweiteter Abschnitt 19 und ein proximaler aufgeweiteter Abschnitt 21 ausgebildet. Zwischen diesen sind jedoch mehrere, im vorliegenden Beispiel zwei mediale aufgeweitete Verbindungsgewindeabschnitte ausgebildet, nämlich ein distaler Verbindungsgewindeabschnitt 28 und ein proximaler Verbindungsgewindeabschnitt 29. Der Innendurchmesser des distalen aufgeweiteten Abschnitts 19 ist kleiner als der des distalen Verbindungsgewindeabschnitts 28, der wiederum kleiner ist als der des proximalen Verbindungsgewindeabschnitts 29, der wiederum kleiner ist als der des proximalen aufgeweiteten Abschnitts 21. Anders ausgedrückt steigt der Innendurchmesser von distal nach proximal an.

Das Schaftkopfelement 2 nach der Erfindung weist distal einen Schaft 23 auf. Dieser ist gestuft ausgebildet, mit seinem distalen Schaftabschnitt 24 und seinem proximalen Schaftabschnitt 26. Zwischen diesen sind jedoch mehrere, im vorliegenden Beispiel zwei mediale aufgeweitete Verbindungsgewindeschaftabschnitte ausgebildet, nämlich ein distaler Verbindungsgewindeschaftabschnitt 30 und ein proximaler Verbindungsgewindeschaftabschnitt 31. Der Innendurchmesser des distalen Schaftabschnitts 24 ist kleiner als der des distalen Verbindungsgewindeschaftabschnitts 30, der wiederum kleiner ist als der des proximalen Verbindungsgewindeschaftabschnitts 31, der wiederum kleiner ist als der des proximalen Schaftabschnitts 26. Anders ausgedrückt steigt der Innendurchmesser von distal nach proximal an.

Die insgesamt vier Verbindungsgewindeabschnitte 28, 29, 30, 31 stehen miteinander in Gewindeeingriff, bilden Verbindungsgewinde 35 bzw. 36 aus und koppeln die Knochenschraube 1 mit dem Schaftkopfelement 2. Genauer gesagt steht der distale Verbindungsgewindeabschnitt 28 mit dem distalen Verbindungsgewindeschaftabschnitt 30 und der proximale Verbindungsgewindeabschnitt 29 mit dem proximalen Verbindungsgewindeschaftabschnitt 31 in Gewindeeingriff. Durch diese Konstruktion ist das Schaftkopfelement 2 von proximal nach distal durch die Aufnahmehülse 3 einsetzbar und kann dann mit der Knochenschraube 1 verschraubt werden, so dass diese mit nahezu beliebigem Nenndurchmesser unabhängig von der Geometrie der Aufnahmehülse 3 ausgebildet sein kann. Im vollständig verschraubten Zustand liegt die Knochenschraube 1 mit ihrer Stirnseite 32 an einem proximal des proximalen Schaftabschnitts 26 ausgebildeten Anschlag 33 an. Das Doppelverbindungsgewinde 28, 29, 30, 31 bewirkt, dass der gesamte Bereich zwischen dem distalen Verbindungsgewinde 28, 30 und dem Anschlag 33 unter Druckspannung gebracht werden kann. Diese Druckspannung bewirkt eine größere Kapazität hinsichtlich einer Aufnahme von ggf. wirkenden biomechanischen, auf das Pedikelschraubensystem wirkenden Kräften. Unter das proximale Verbindungsgewinde 28, 30 würde die Schraube wie vorstehend bei Stand der Technik anhand der Figur 1 beschrieben eine bevorzugte Versagensstelle aufweisen und dort bevorzugt brechen. Durch die erfindungsgemäße mehrfache, feinere Abstufung der Durchmesser kann Kerbwirkung in vorteilhafter Weise reduziert und minimiert werden.

Die Knochenschraube 1 weist an ihrem proximalen aufgeweiteten Abschnitt 21 einen Torxprofilaufnahmeabschnitt 37 auf. Dieser Torxprofilaufnahmeabschnitt 37 ermöglicht die Revision der Knochenschraube 1, falls sich das Schaftkopfelement 2 von der Knochenschraube 1 getrennt hat.

### Bezugszeichenliste

- 1: Knochenschraube
- 2: Schaftkopfelement
- 3: Aufnahmehülse
- 4: Klemmstempel
- 5: Durchgangsöffnung
- 6: Kugelkopf
- 7: Aufnahmeraum
- 8: Hülsenwandabschnitt
- 9: Hülsenwandabschnitt
- 10: Innengewinde
- 11: Werkzeugaufnahme
- 12: Aufnahmefläche
- 13: Lagerschale
- 14: Durchgangsöffnung
- 15: Werkzeugaufnahme
- 16: Durchgangsöffnung
- 17: Durchgangsöffnung
- 18: Knochengewinde
- 19: distaler aufgeweiteter Abschnitt
- 20: medialer aufgeweiteter Abschnitt
- 21: proximaler aufgeweiteter Abschnitt
- 22: Verbindungsgewinde (der Knochenschraube)
- 23: Schaft, Zapfen
- 24: distaler Schaftabschnitt
- 25: medialer Schaftabschnitt
- 26: proximaler Schaftabschnitt
- 27: Verbindungsgewinde (des Schaftkopfelements)
- 28: distaler Verbindungsgewindeabschnitt (der Knochenschraube)
- 29: proximaler Verbindungsgewindeabschnitt (der Knochenschraube)
- 30: distaler Verbindungsgewindeschaftabschnitt (des Schaftkopfelements)
- 31: proximaler Verbindungsgewindeschaftabschnitt (des Schaftkopfelements)
- 32: Stirnseite
- 33: Anschlag
- 34: Kugelaufnahme
- 35: Verbindungsgewinde
- 36: Verbindungsgewinde
- 37: Torxprofilaufnahmeabschnitt

## Patentansprüche

1. Pedikelschraubensystem mit einer Aufnahmehülse (3) für einen Längsträger, einer Knochenschraube (1) und einem Schaftkopfelement (2),
wobei die Knochenschraube (1) ein als Außengewinde ausgebildetes Knochengewinde (18) und ein als Innengewinde ausgebildeten Verbindungsgewinde (22) aufweist, und
wobei das Schaftkopfelement (2) proximal einen Kopf (6), auf dem die Aufnahmehülse (3) gelagert ist, und distal ein als Außengewinde ausgebildetes Verbindungsgewinde (27) aufweist und damit in das Verbindungsgewinde (22) der Knochenschraube (1) eingeschraubt ist,
**dadurch gekennzeichnet, dass**
die Verbindungsgewinde (22, 27) jeweils als Mehrfachgewinde mit jeweils einem distalen Verbindungsgewindeabschnitt (28, 30) und einem proximalen Verbindungsgewindeabschnitt (29, 31) ausgebildet sind, wobei
der distale Verbindungsgewindeabschnitt (28, 30) und der proximale Verbindungsgewindeabschnitt (29, 31) eine identische Gewindesteigung aufweisen; und der distale Verbindungsgewindeabschnitt (28, 30) einen kleineren Durchmesser als der proximale Verbindungsgewindeabschnitt (29, 31) aufweist.

2. Pedikelschraubensystem nach Anspruch 1, **dadurch gekennzeichnet, dass** die Verbindungsgewinde der Knochenschraube (1) als Doppelgewinde ausgebildet sind, mit einem ersten, distalen Verbindungsgewindeabschnitt (28) und einem zweiten, proximalen Verbindungsgewindeabschnitt (29), und die Verbindungsgewinde des Schaftkopfelements (2) als Doppelgewinde ausgebildet sind, mit einem ersten, distalen Verbindungsgewindeabschnitt (30) und einem zweiten, proximalen Verbindungsgewindeabschnitt (31).

3. Pedikelschraubensystem nach Anspruch 1 oder 2, **dadurch gekennzeichnet, dass** der Gewindedurchmesser und die Gewindesteigung jedes Verbindungsgewindeabschnitts (28, 29, 30, 31) jeweils konstant sind.

4. Pedikelschraubensystem nach einem der vorstehenden Ansprüche, **dadurch gekennzeichnet, dass** das Schaftkopfelement (2) einen Anschlag (33) aufweist, an dem eine Anschlagfläche der Knochenschraube (1), insbesondere deren proximale Stirnfläche (32) anliegt, wenn das Schaftkopfelement (2) und die Knochenschraube (1) über ihre jeweiligen Verbindungsgewinde (28, 29, 30, 31) vollständig miteinander verschraubt sind.

5. Pedikelschraubensystem nach einem der vorstehenden Ansprüche, **dadurch gekennzeichnet, dass** die Knochenschraube (1) eine sich in Richtung der Längsachse erstreckende zentrale, gestufte Öffnung (17) aufweist, insbesondere in Form einer Sackbohrung oder einer Durchgangsbohrung (17), mit Abschnitten (19, 21, 28, 29) unterschiedlichen Innendurchmessers.

6. Pedikelschraubensystem nach Anspruch 5, **dadurch gekennzeichnet, dass** die zentrale Öffnung (17) der Knochenschraube (1) einen proximalen gewindelosen oder mit einem Torxinnenprofil (37) versehenen Abschnitt (21) und distalen gewindelosen Abschnitt (19) aufweist, zwischen denen der distale Verbindungsgewindeabschnitt (28) und der proximale Verbindungsgewindeabschnitt (29) angeordnet sind.

7. Pedikelschraubensystem nach einem der vorstehenden Ansprüche, **dadurch gekennzeichnet, dass** das Schaftkopfelement (2) einen gestuften Zapfen (23) aufweist, mit einem proximalen gewindelosen Abschnitt (26) und einem distalen gewindelosen Abschnitt (24), zwischen denen der distale Verbindungsgewindeabschnitt (30) und der proximale Verbindungsgewindeabschnitt (31) angeordnet sind.

8. Pedikelschraubensystem nach Anspruch 6 oder 7, **dadurch gekennzeichnet, dass** der distale gewindelose Abschnitt (24) einen kleineren Durchmesser aufweist als der distale Verbindungsgewindeabschnitt (30) und dass der proximale gewindelose Abschnitt (26) einen größeren Durchmesser aufweist als der proximale Verbindungsgewindeabschnitt (31).

9. Pedikelschraubensystem nach einem der vorstehenden Ansprüche, **dadurch gekennzeichnet, dass** das Schaftkopfelement (2) eine sich in Richtung der Längsachse erstreckende zentrale Öffnung (16) aufweist, insbesondere in Form einer Durchgangsbohrung (16).

10. Pedikelschraubensystem nach einem der vorstehenden Ansprüche, **dadurch gekennzeichnet, dass** das Knochengewinde (18) einen zylinderförmigen Gewindekern mit im Wesentlichen konstantem Kerndurchmesser aufweist.

11. Pedikelschraubensystem nach einem der vorstehenden Ansprüche, **dadurch gekennzeichnet, dass** die Gewindetiefe des Knochengewindes (18) im Bereich der Verbindungsgewinde (28, 29) konstant ist.

## Claims

1. Pedicle screw system comprising a receiving sleeve (3) for a longitudinal beam, a bone screw (1) and a shank head element (2),
wherein the bone screw (1) has a bone thread (18) formed as an external thread and a connection thread (22) formed as an internal thread, and
wherein the shank head element (2) proximally has a head (6) on which the receiving sleeve (3) is mounted, and distally has a connection thread (27) formed as an external thread by which it is screwed into the connection thread (22) of the bone screw (1),
**characterised in that**
the connection threads (22, 27) are each formed as multiple threads each having a distal connection thread portion (28, 30) and a proximal connection thread portion (29, 31), wherein
the distal connection thread portions (28, 30) and the proximal connection thread portions (29, 31) have an identical thread pitch; and the distal connection thread portions (28, 30) have a smaller diameter than the proximal connection thread portions (29, 31).

2. Pedicle screw system according to claim 1, **characterised in that** the connection thread of the bone screw (1) is formed as a double thread, with a first, distal connection thread portion (28) and a second, proximal connection thread portion (29), and the connection thread of the shank head element (2) is formed as a double thread, with a first, distal connection thread portion (30) and a second, proximal connection thread portion (31).

3. Pedicle screw system according to claim 1 or 2, **characterised in that** the thread diameter and the thread pitch of each connection thread portion (28, 29, 30, 31) are each constant.

4. Pedicle screw system according to any one of the preceding claims, **characterised in that** the shank head element (2) has a stop (33) on which abuts a stop surface of the bone screw (1), in particular its proximal end face (32), when the shank head element (2) and the bone screw (1) are completely screwed together via their respective connection threads (28, 29, 30, 31).

5. Pedicle screw system according to any one of the preceding claims, **characterised in that** the bone screw (1) has a central, stepped opening (17) extending in the direction of the longitudinal axis, in particular in the form of a blind bore or a through bore (17), with portions (19, 21, 28, 29) of differing internal diameter.

6. Pedicle screw system according to claim 5, **characterised in that** the central opening (17) of the bone screw (1) has a proximal portion (21) that is threadless or has a Torx inner profile (37) and a distal threadless portion (19), between which are arranged the distal connection thread portion (28) and the proximal connection thread portion (29).

7. Pedicle screw system according to any one of the preceding claims, **characterised in that** the shank head element (2) comprises a stepped pin (23) having a proximal threadless portion (26) and a distal threadless portion (24), between which the distal connection thread portion (30) and the proximal connection thread portion (31) are arranged.

8. Pedicle screw system according to claim 6 or 7, **characterised in that** the distal threadless portion (24) has a smaller diameter than the distal connection thread portion (30) and the proximal threadless portion (26) has a larger diameter than the proximal connection thread portion (31).

9. Pedicle screw system according to any one of the preceding claims, **characterised in that** the shank head element (2) has a central opening (16) extending in the direction of the longitudinal axis, in particular in the form of a through bore (16).

10. Pedicle screw system according to any one of the preceding claims, **characterised in that** the bone thread (18) has a cylindrical thread core with a substantially constant core diameter.

11. Pedicle screw system according to any one of the preceding claims, **characterised in that** in the region of the connection threads (28, 29) the thread depth of the bone thread (18) is constant.

## Revendications

1. Système de vis pédiculaire avec un manchon de logement (3) pour un support longitudinal, avec une vis d'ostéosynthèse (1) et avec un élément tête de tige (2),
dans lequel la vis d'ostéosynthèse (1) présente un filetage côté os (18) réalisé comme un filetage extérieur et un filetage de liaison (22) réalisé comme un filetage intérieur, et dans lequel l'élément tête de tige (2) présente du côté proximal une tête (6) sur laquelle est monté le manchon de logement (3) et du côté distal un filetage de liaison (27) qui est réalisé comme un filetage extérieur et il est ainsi vissé dans le filetage de liaison (22) de la vis d'ostéosynthèse (1),
**caractérisé en ce que**
les filetages de liaison (22, 27) sont réalisés à chaque fois comme des filetages multiples avec chacun un tronçon fileté de liaison distal (28, 30) et un tronçon fileté de liaison proximal (29, 31),
lequel tronçon fileté de liaison distal (28, 30) et lequel tronçon fileté de liaison proximal (29, 31) présentent un pas de filetage identique,
et le tronçon fileté de liaison distal (28, 30) présente un plus petit diamètre que le tronçon fileté de liaison proximal (29, 31).

2. Système de vis pédiculaire selon la revendication 1, **caractérisé en ce que** le filetage de liaison de la vis d'ostéosynthèse (1) est réalisé comme un double filetage, avec un premier tronçon fileté de liaison distal (28) et un deuxième tronçon fileté de liaison proximal (29), et le filetage de liaison de l'élément tête de tige (2) est réalisé comme un double filetage, avec un premier tronçon fileté de liaison distal (30) et un deuxième tronçon fileté de liaison proximal (31).

3. Système de vis pédiculaire selon la revendication 1 ou 2, **caractérisé en ce que** le diamètre de filetage et le pas de filetage de chaque tronçon filet de liaison (28, 29, 30, 31) sont tous constants.

4. Système de vis pédiculaire selon l'une quelconque des revendications précédentes, **caractérisé en ce que** l'élément tête de tige (2) présente une butée (33) contre laquelle s'appuie une surface de butée de la vis d'ostéosynthèse (1), en particulier sa surface frontale proximale (32), lorsque l'élément tête de tige (2) et la vis d'ostéosynthèse (1) sont complètement vissés ensemble par l'intermédiaire de leurs filetages de liaison respectifs (28, 29, 30, 31).

5. Système de vis pédiculaire selon l'une quelconque des revendications précédentes, **caractérisé en ce que** la vis d'ostéosynthèse (1) présente une ouverture (17) centrale à gradins, qui s'étend dans la direction de l'axe longitudinal, en particulier sous la forme d'un trou borgne ou d'un trou traversant (17), avec des tronçons (19, 21, 28, 29) de différents diamètres.

6. Système de vis pédiculaire selon la revendication 5, **caractérisé en ce que** l'ouverture centrale (17) de la vis d'ostéosynthèse (1) présente un tronçon proximal (21) sans filetage ou muni d'un profil intérieur du type Torx (37) et un tronçon distal (19) sans filetage, entre lesquels sont agencés le tronçon fileté de liaison distal (28) et le tronçon fileté de liaison proximal (29).

7. Système de vis pédiculaire selon l'une quelconque des revendications précédentes, **caractérisé en ce que** l'élément tête de tige (2) présente un pivot à gradins (23), avec un tronçon proximal (26) sans filetage et un tronçon distal (24) sans filetage, entre lesquels sont agencés le tronçon fileté de liaison distal (30) et le tronçon fileté de liaison proximal (31).

8. Système de vis pédiculaire selon la revendication 6 ou 7, **caractérisé en ce que** le tronçon distal sans filetage (24) présente un plus petit diamètre que le tronçon fileté de liaison distal (30) et **en ce que** le tronçon proximal sans filetage (26) présente un plus grand diamètre que le tronçon fileté de liaison proximal (31).

9. Système de vis pédiculaire selon l'une quelconque des revendications précédentes, **caractérisé en ce que** l'élément tête de tige (2) présente une ouverture centrale (16), qui s'étend dans la direction de l'axe longitudinal, en particulier sous la forme d'un trou traversant (16).

10. Système de vis pédiculaire selon l'une quelconque des revendications précédentes, **caractérisé en ce que** le filetage côté os (18) présente un noyau de filetage cylindrique avec un diamètre de noyau sensiblement constant.

11. Système de vis pédiculaire selon l'une quelconque des revendications précédentes, **caractérisé en ce que** la profondeur de filetage du filetage côté os (18) est constante dans la zone du filetage de liaison (28, 29).
